# FASCICULE DE BREVET EUROPEEN

(11) **EP 3 047 463 B1**
(45) Date de publication et mention de la délivrance du brevet: **25.11.2020**
(21) Numéro de dépôt: 14784296.7
(22) Date de dépôt: 16.09.2014
(51) Int. Cl.: A61B 10/00, G07F 17/00, G07F 11/62

(54) **PROCÉDÉ DE DÉTECTION DE LA POSITION D'UNE CASSETTE ET SON DISPOSITIF**
VERFAHREN ZUR ERKENNUNG EINER KASSETTENPOSITION UND VORRICHTUNG DAFÜR
METHOD FOR DETECTING THE POSITION OF A CASSETTE AND DEVICE THEREFOR

(30) Priorité: 17.09.2013 FR 1358925
(43) Date de publication de la demande: 27.07.2016
(73) Titulaire: Dreampath Diagnostics, 67000 Strasbourg (FR)
(72) Inventeur: WILHELM, Valérie, F-67114 Eschau (FR); JORDAN NAVAS, Pablo, 2805 MADRID (ES)
(74) Mandataire: Hugues, Catherine
(86) Numéro de dépôt international: PCT/FR2014/052297
(87) Numéro de publication internationale: WO 2015/040320

(56) Documents cités:
- WO-A1-2010/004331
- DE-U1- 9 416 270
- FR-A1- 2 985 590
- US-A1- 2007 135 965
- US-A1- 2007 185 615

## Description

La présente invention entre dans le domaine médical de l'analyse de prélèvements tissulaires et cellulaires.

L'invention concerne plus particulièrement la conservation et le stockage de tels prélèvements.

Dans le cadre de la prise en charge médicale d'un patient ou dans le cadre de la recherche, des prélèvements tissulaires ou cellulaires peuvent être effectués en vue d'une analyse histologique et/ou moléculaire. A des fins de conservation, ces éléments sont déshydratés puis conservés inclus en paraffine dans un support appelé communément « cassettes ». Après réalisation des coupes nécessaires à l'analyse histologique et/ou moléculaire, les résidus tissulaire et cellulaire inclus en paraffine sont conservés afin de pouvoir accéder ultérieurement à des analyses complémentaires (parfois des années plus tard).

De telles cassettes sont généralement constituées d'un boîtier de forme standardisée parallélépipédique rectangle, avec ou sans couvercle, dont le fond est ajouré par des orifices traversants. De plus, une des parois latérales, généralement la paroi avant, reçoit des informations uniques d'identification de l'échantillon, telle une référence. Une telle paroi peut être prévue inclinée, afin de faciliter la lecture desdites informations.

Plusieurs exemples de telles cassettes sont décrits au travers des documents US D448 487 S, US 4 421 246 ou GB 2 113 249.

Ces cassettes et leur échantillon sont répertoriés et stockés par différents établissements, notamment les laboratoires d'analyse médicale. Légalement, leur conservation est obligatoire pour chaque individu sur une durée de dix années minimum pour une entreprise privée, allant jusqu'à plusieurs décennies pour un établissement public ou une industrie pharmaceutique.

Une telle cassette présente généralement des dimensions standardisées, avec une hauteur de 41,8 millimètres (mm), pour 28,5 mm de large et 6,5 mm de hauteur ou d'épaisseur.

A l'heure actuelle, lesdites cassettes sont rangées et manipulées manuellement, par des opérateurs n'ayant pas forcément reçu de formation spécifique, sans aucun contrôle. De ce fait, une telle gestion manuelle entraine des erreurs et une perte de temps considérable dans leur recherche, allant jusqu'à la perte préjudiciable de certains prélèvements.

De plus, dans ce contexte, les quantités de cassettes existantes sont en constante évolution : d'environ 200 millions par an dans les années 1990, leur nombre a augmenté à près de 400 millions par an en 2010 et cette croissance est estimée à 750 millions par an vers 2030. Dès lors, les capacités de stockage et les moyens empiriques mis en œuvre actuellement ne sont pas prévus pour supporter la gestion d'un stockage rationnel et sûr pour de telles quantités.

De ce fait, il a été imaginé un système d'ordonnancement informatisé et automatisé de cassettes de prélèvement biologique. Un tel système se veut à même d'offrir une traçabilité et une gestion rationnelle de cassettes, pour des quantités importantes, notamment réparties dans des lieux géographiques distincts.

Pour ce faire, un tel système prévoit tout d'abord des moyens de stockage de plusieurs cassettes, par positionnement au sein d'au moins un compartiment de stockage horizontal, sous forme d'un tiroir. Ce dernier présente des logements constitués de parois verticales s'étendant selon toute la largeur du tiroir, créant plusieurs colonnes ainsi séparées. La largeur des colonnes correspond à celle des cassettes à y introduire. De plus, chaque colonne est subdivisée sur toute sa longueur en compartiments, séparés par des ergots ménagés en vis-à-vis les uns des autres de façon saillante orthogonalement par rapport à chaque paroi, s'étendant vers l'intérieur de ladite colonne. De plus, lesdits ergots sont espacés complémentairement à l'épaisseur d'une cassette. Chaque compartiment permet alors de recevoir une cassette.

Ainsi, le positionnement des cassettes au sein dudit tiroir s'effectue selon des colonnes et rangées. De plus, lesdites cassettes y sont disposées verticalement, face avant comportant des informations d'identification tournées vers le haut. De telles informations sont prévues uniques pour chaque cassette. Elles peuvent être enregistrées au sein d'un système de gestion, assurant leur traçabilité, ainsi que leur référencement en correspondance avec l'échantillon de tissu que chaque cassette enferme, en outre le dossier médical de son patient.

De plus, ces informations d'identification se présentent sous la forme d'un codage optique, notamment sous la forme d'un code à barres ou matriciel, destiné à être lu par un lecteur optique, en particulier sous forme d'un scanner lumineux.

Un tel système présente l'avantage de ne faire intervenir aucun ordonnancement au moment du rangement desdites cassettes au sein d'un tel stockage, les cassettes pouvant être positionnées sans ordre précis de rangement, lors du remplissage des colonnes et rangées. Le caractère d'ordonnancement découle de ce stockage particulier au travers de moyens automatiques de lecture desdites informations présentes sur chaque cassette et de référencement informatisé de la position de ladite cassette au sein des moyens de stockage.

Ce système de rangement de cassettes sous forme de tiroir et d'ordonnancement desdites cassettes est décrit dans le document FR 2 985 590, n'abordant que de façon globale, le principe de rangement et de détection, sans envisager de technique spécifique et optimale de détection.

L'invention concerne tout particulièrement le fonctionnement automatique spécifique de la lecture et du référencement des cassettes rangées au sein d'un tel tiroir. De façon essentielle, l'invention vise à effectuer plusieurs reconnaissances successives, pour déterminer la présence, la position et identifier les cassettes présentes. Ces différentes identifications sont réitérées en cas d'erreur survenues dans la détection, avec une trajectoire spécifique, de manière à optimiser le temps nécessaire à une telle opération de scannage.

Pour ce faire, l'invention concerne un procédé de référencement assurant, d'une part, la détection systématique de la présence ou de l'absence de toute cassette enfichée au sein des compartiments dudit tiroir, d'autre part, la lecture des données présentes sous forme de code sur chaque cassette détectée et, d'autre part encore, le référencement automatique de la position de chaque cassette détectée par l'intermédiaire des coordonnées du compartiment au sein duquel elle est insérée.

En outre, la détection et la lecture s'effectuent de façon optique, par une mire optique et par un scanner lumineux prévus solidaires, mobiles relativement par rapport audit tiroir, et assurant la lecture des codes présents sur chaque cassette, en vue de son identification et de sa mise en correspondance avec sa position bidimensionnelle.

Plus particulièrement, ledit procédé comprend les étapes suivantes :
- on positionne au moins une cassette enfichée la face supérieure tournée vers le haut au sein d'un compartiment dudit tiroir ;
- on effectue automatiquement, lors d'un premier passage, une détection systématique de tous les compartiments par vérification de la présence d'une cassette ;
- on effectue automatiquement une détection systématique de tous les compartiments par vérification de la présence d'une cassette au sein de chacun desdits compartiments, la détection étant optique et consistant à déplacer automatiquement relativement par rapport au-dessus dudit tiroir selon au moins une trajectoire passant par tous les compartiments des moyens de détection et de lecture comprenant une mire optique (3) émettant un faisceau lumineux, notamment un faisceau laser, permettant de mesurer la distance entre son point d'émission et le point de réflexion, et un scanner lumineux;
- en cas de détection de la présence d'une cassette au sein de son compartiment, on effectue une lecture, par le scanner lumineux de données présentes sur ladite face supérieure et on identifie ladite cassette ;
- on référence et on enregistre la position bidimensionnelle de ladite cassette identifiée par l'intermédiaire des coordonnées de son compartiment, selon sa colonne et sa rangée ;
- en cas d'erreur lors de la lecture par le scanner lumineux, on enregistre la position où l'erreur de lecture a été constatée,

Un tel procédé se caractérise en ce que :
- en cas d'erreur de lecture lors d'un premier passage, les moyens de détection et de lecture sont déplacés directement vers la position où l'erreur survenue a été constatée et préalablement enregistrée, sans opérer une nouvelle détection et/ou une lecture de tous les autres compartiments où ces opérations se sont déroulées convenablement auparavant lors du premier passage, et la détection est réitérée directement à l'emplacement où l'erreur est survenue, s'abstenant de refaire l'intégralité du premier passage qui parcourt l'intégralité du tiroir.

Selon une caractéristique additionnelle, nullement limitative, la lecture peut être optique, lesdites données pouvant être encodées sous forme d'un code à barres ou matriciel lu par le scanner lumineux.

De préférence, le référencement peut comprendre une étape d'enregistrement de la position référencée en rapport avec les données de chaque cassette au sein d'un gestionnaire.

Selon un mode de réalisation spécifique, ledit procédé peut consister à effectuer plusieurs détections et lectures successives des compartiments dudit tiroir.

D'autres caractéristiques et avantages de l'invention ressortiront de la description détaillée qui va suivre des modes de réalisation non limitatifs de l'invention, en référence aux figures annexées, dans lesquelles :
- la figure 1 représente schématiquement une vue en perspective d'un dispositif de mise en œuvre du procédé selon l'invention, lors de l'introduction ou de l'extraction d'un tiroir ;
- la figure 2 représente schématiquement une vue similaire à la figure 1, dans laquelle le carter de protection dudit dispositif a été retiré, laissant apparaître les moyens de lecture et de référencement des cassettes présentes au sein dudit tiroir ; et
- la figure 3 représente schématiquement une vue de côté d'un détail des moyens de détection des cassettes et de lecture optique des informations d'identification, au cours d'une étape du procédé selon l'invention.

La présente invention concerne la détection et le référencement de la position bidimensionnelle d'au moins une cassette 1, de préférence plusieurs, au sein d'un tiroir 2.

Comme évoqué précédemment, de telles cassettes 1 se présentent sous la forme de blocs creux, destinés à recevoir un échantillon englobé de paraffine. Ce bloc présente une forme parallélépipédique rectangle, avec une paroi présentant un champ incliné, en biseau. Ce chant est destiné à être positionné vers le haut, lors du rangement d'une cassette 1 au sein d'un tiroir 2.

A ce titre, ledit tiroir 2 comporte des compartiments 22 répartis selon des colonnes 23 et des rangées 24.

Selon le mode de réalisation préférentielle, comme représenté sur les figures, afin de permettre le positionnement vertical des cassettes 1, facilitant leur identification une fois en place, chaque tiroir 2 présente au niveau de son fond des moyens d'emboîtement. Ces derniers sont ménagés sous forme de saillies ou redents 20, espacés régulièrement, ménageant ainsi des parties mâles et femelles au niveau dudit fond, au sein desquelles peuvent être enfichées lesdites cassettes 1. En somme, l'espace entre chaque saillie ou redent 20 constitue un logement ou une fente de réception en emboîtement d'une cassette 1.

Selon le mode préférentiel de réalisation, lesdits redents 20 sont ménagés en saillie au niveau du fond, à savoir en partie inférieure du tiroir 2, mais le long et de part et d'autre de parois intérieures verticales 21. Ces dernières sont orientées parallèlement, à intervalles réguliers, de manière à définir des colonnes 23 au sein dudit tiroir 2.

On notera que l'espacement entre les saillies ou redents 20 est dimensionné de manière à autoriser l'emboîtement de chaque cassette 1, avec ou sans jeu, préférentiellement sans jeu. Dans ce dernier cas, la largeur de chaque espacement, à savoir la distance entre les surfaces en vis-à-vis de deux saillies ou redents 20 consécutifs, est quasiment égale à l'épaisseur d'une cassette 1, de un à plusieurs dixièmes de millimètres près, offrant un jeu minime.

Selon l'exemple de cassettes évoquées en partie introductive, la largeur d'une rangée 24 peut être d'environ 29 millimètres (mm), de préférence 29,2 mm pour un bloc de 28,5 mm de large. L'espacement entre les saillies ou redents 20 peut alors être d'environ 6 mm, de préférence 6,6 mm, pour un bloc de 6,6 mm d'épaisseur. Enfin, la hauteur du tiroir peut être d'environ 50 à 60 mm, de préférence 53,5 mm, pour une cassette d'une longueur de 41,8 mm, tandis que la hauteur des parois médianes 21 peut être inférieure, notamment d'au moins 10 à 30 mm, laissant ainsi sortir le haut des cassettes 1 permettant de faciliter leur préhension.

En outre, l'épaisseur desdites saillies, ergots ou redents 20 permet la préhension et l'extraction, manuelle ou automatique, notamment robotisée, des cassettes 1. Pour ce faire, selon un mode particulier de réalisation, lesdites saillies ou redents 20 peuvent s'étendre uniquement sur une partie de la hauteur de chaque paroi médiane intérieure 21. De préférence, la partie supérieure de chaque paroi 21 est dépourvue de redents 20, ces derniers ne s'étendant que depuis le fond sur une hauteur moindre que celle des parois latérales 21 qui l'entourent, à savoir deux parois 21 ou bien les bords latéraux dudit tiroir 2.

Avantageusement, l'invention prévoit de détecter et de référencer la position bidimensionnelle de toute cassette 1 se trouvant enfichée au sein des compartiments 22 d'un tel tiroir 2.

Pour ce faire, tout d'abord, on positionne au moins une cassette 1, de préférence plusieurs, enfichée la face supérieure tournée vers le haut au sein d'un compartiment 22 dudit tiroir 2.

On notera que ce positionnement s'effectue de façon arbitraire, n'importe quelle cassette 1 pouvant être positionnée au niveau de n'importe quel compartiment 22 libre, à savoir vide de toute cassette. Il n'est donc pas nécessaire de remplir toutes les colonnes 23 et toutes les rangées 24. Aucun ordre de rangement n'est requis.

Par conséquent, sans classement préalable, il est nécessaire d'identifier chaque cassette 1 et de calculer sa position au sein du tiroir 2.

Pour ce faire, dans un premier temps, on effectue automatiquement une détection systématique de tous les compartiments 22 par vérification de la présence d'une cassette 1 au sein de chacun desdits compartiments 22. En somme, un balayage est opéré pour vérifier si un compartiment 22 comprend une cassette 1 ou non.

Pour ce faire, selon le mode préférentiel de réalisation, on déplace relativement par rapport au dessus dudit tiroir 2, une mire optique 3. Cette dernière émet un faisceau lumineux 30, notamment un faisceau laser, permettant de mesurer la distance entre son point d'émission et le point de réflexion. En somme, si le laser rencontre le fond du compartiment 22 ou bien la face supérieure d'une cassette 1, alors la distance mesurée sera différente. Ladite mire 3 ou bien des moyens connexes permettent ainsi de déduire la présence ou l'absence d'une cassette 1 au sein d'un compartiment 22, à partir de la distance ainsi mesurée.

Puis, dans un second temps, en cas de détection de la présence d'une cassette 1 au sein de son compartiment 22, on effectue une lecture de données présentes sur ladite face supérieure et on identifie ladite cassette 1.

A ce titre, la lecture est prévue optique, lesdites données étant alors encodées sous forme d'un code à barres ou matriciel lu par un scanner lumineux 4. Ce dernier permet d'émettre un faisceau lumineux 40 et de recevoir un faisceau réfléchi, permettant de déterminer le code présent sur chaque cassette 1.

On notera que cette lecture s'effectue simultanément avec l'étape de détection, à savoir d'affilée compartiment 22 par compartiment 22. De plus, la lecture peut s'effectuer par émission et réception du faisceau lumineux en continu ou à intervalles temporels réguliers, notamment entre des laps de temps correspondant au déplacement relatif d'un compartiment 22 à un autre.

Selon une caractéristique spécifique, le déplacement de la mire 3 et du scanner 4 peut s'effectuer de façon relative par rapport audit tiroir 2. En d'autres termes, selon le mode préférentiel de réalisation, ils peuvent se déplacer par rapport au tiroir 2, mais selon un autre mode de réalisation envisagé, c'est le tiroir 2 qui peut se déplacer par rapport à la mire 3 et au scanner 4, ces derniers restant fixes.

De plus, la mire 3 et le scanner 4 peuvent être solidaires l'un de l'autre, se déplaçant relativement ensemble. Selon un autre mode de réalisation envisagé, la mire 3 et le scanner 4 peuvent se déplacer de façon distincte.

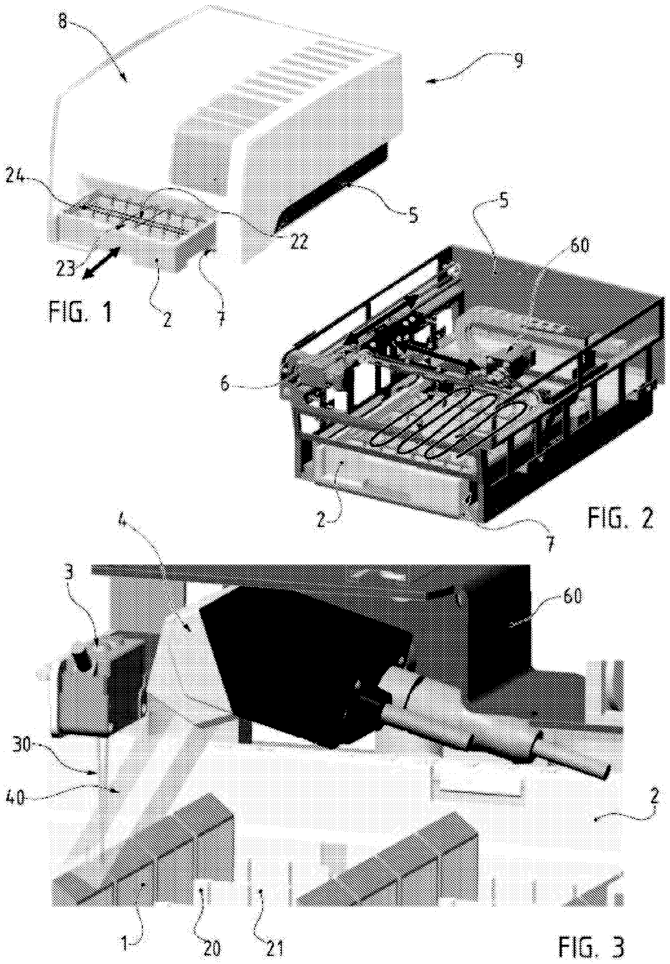

A ce titre, dans le mode de réalisation préférentiel, comme visible sur la figure 2, la mire 3 et le scanner 4 sont montés conjointement mobiles par rapport à une structure fixe 5. Pour ce faire, cette dernière comporte un châssis recevant supérieurement des moyens 6 de déplacement et d'entraînement des moyens de détection et de lecture, à savoir de la mire 3 et du scanner 4. Ces moyens 6 comprennent notamment un chariot 7 mobile supportant ladite mire 3 et le scanner 4. Un tel chariot 7 est mobile et entraîné en translation selon au moins deux degrés d'orientations, comme schématisé par les flèches visibles sur la figure 2.

En outre, ce déplacement s'effectue au dessus du tiroir 2, au niveau de sa face ouverte, vers laquelle sont tournées les cassettes 1 avec leur face pourvue des données d'identification vers le haut.

En particulier, le déplacement peut suivre une trajectoire précise. Cette dernière peut consister à partir d'un point situé de préférence à un angle de la structure 5, pour venir parcourir chaque rangée 24 ou colonne 23 de compartiments 22 les uns après les autres, dans un sens comme dans l'autre. En particulier, cette trajectoire peut suivre les colonnes 23 depuis l'avant vers l'arrière puis passer à la colonne 23 adjacente depuis l'arrière vers l'avant, et ainsi de suite jusqu'à la dernière colonne 23. Un tel exemple de trajectoire est schématisé sur la figure 2. Une fois cette trajectoire parcourue, formant un cheminement, les moyens de détection sont renvoyés à la position d'origine.

En outre, de façon essentielle, plusieurs cheminements successifs sont envisagés, notamment au moins deux mais préférentiellement au nombre de trois. Ce double ou triple passage permet de s'assurer de la lecture systématique de toutes les cassettes 1 et de rectifier les erreurs de détection et de lecture survenues au passage précédent.

On notera alors que le premier cheminement consiste à parcourir une trajectoire couvrant l'intégralité du tiroir 2, à savoir passant au-dessus de chaque compartiment, qu'il comprenne une cassette 1 ou non.

En effet, en cas d'erreur de détection ou de lecture, les moyens de détection et de lecture peuvent être déplacés directement vers la position où l'erreur survenue a été constatée et préalablement enregistrée. En somme, lors d'un premier passage, une erreur survient, comme par exemple la détection d'une cassette 1 au sein d'un compartiment 22 mais l'impossibilité de lire ses données. La position est enregistrée pour, au passage suivant, que les moyens soient déplacés vers cet emplacement en premier, sans opérer une nouvelle détection et/ou une lecture de tous les autres compartiments 22 où ces opérations se sont déroulées convenablement auparavant, lors du passage précédent.

Ainsi, l'invention permet de s'assurer de la bonne détection et identification des cassettes présentes au sein du tiroir, avec un cheminement optimal, même répété. En particulier, en cas d'erreur de lecture, la détection est réitérée directement à l'emplacement où l'erreur est survenue, s'abstenant de refaire l'intégralité du cheminement préalable, en particulier du premier cheminement qui parcourt l'intégralité du tiroir.

A ce titre, les déplacements relatifs des moyens de détection et de lecture peuvent s'opérer en fonction du tiroir 2, à savoir en fonction de pas correspondants à l'écartement entre deux compartiments 22 d'une même colonne 23 ou entre deux rangées 24, ainsi qu'aux extrémités de ces dernières, selon un autre pas correspondant à l'espacement réciproquement entre deux colonnes 23 ou deux compartiments 22. Ces pas sont donc déterminés et spécifiques aux dimensions propres dudit tiroir 2.

De plus, ce sont ces pas qui permettent auxdits moyens de détection et de lecture de savoir la position exacte, selon un repère en deux dimensions.

Selon un autre mode de réalisation envisagé, la position peut être détectée, notamment par lecture au niveau dudit tiroir 2, ce dernier comprenant alors un code pouvant être lu et correspondant à la position bidimensionnelle selon une colonne 23 et une rangée 24.

Dans un cas comme dans l'autre, ces coordonnées comprennent au moins deux variables, comme par exemple selon la forme suivante : A1, A2..., B1, B2... où les lettres A,B... sont affectées aux colonnes 23 successives (ou bien les rangées 24) et les chiffres 1,2... sont affectés aux rangées 24 successives (ou bien réciproquement les colonnes 23).

Une troisième variable peut être ajoutée, correspondant à la référence d'un tiroir 2, chaque tiroir 2 étant alors identifié de manière unique par un numéro propre, qu'il est aussi possible de détecter et de lire avec l'invention.

Une fois la lecture d'une cassette 1 effectuée, on référence la position bidimensionnelle de ladite cassette ainsi identifiée par l'intermédiaire des coordonnées de son compartiment 22, selon sa colonne 23 et sa rangée 24. Ce référencement peut comprendre au moins une étape d'enregistrement de la position référencée en rapport avec les données de chaque cassette 1 au sein d'un gestionnaire.

Ce dernier peut être connecté directement, ou bien à distance, avec le système selon l'invention. Il permet notamment d'afficher en temps réel la détection et la lecture des cassettes 1 présentes au sein d'un tiroir 2. En particulier, en cas d'erreur, par exemple de non lecture des données, le compartiment 22 peut être représenté sur un codage en corrélation, notamment de couleur rouge. Dans le même état d'esprit, si une détection et une lecture s'est bien déroulée, le compartiment 22 comprend une autre couleur, comme vert, ainsi que les données uniques d'identification de la cassette 1 qui s'y trouve.

En outre, un tel gestionnaire peut être constitué d'une base de données relationnelle, permettant d'assurer le stockage de toutes les données enregistrées, que ce soient les cassettes 1 identifiées et leur position référencée, mais aussi toute donnée liée aux opérations effectuées, comme par exemple les statistiques d'utilisation du système, les erreurs survenues, le nombre de cycles et de passages, etc.

Ce gestionnaire offre ainsi une traçabilité parfaite, accessible localement ou à distance, permettant de connaître l'emplacement exact d'une cassette 1.

De plus, à la fin d'un cycle total de référencement, une photographie numérique de chaque tiroir 2 peut être opérée. Selon une autre possibilité, une photographie numérique de chaque cassette 1 peut être effectuée au moment de sa lecture.

Pour ce faire, l'invention prévoit d'embarquer, avec les moyens de détection et de lecture, une caméra numérique, dont l'objectif est tourné vers la face supérieure ouverte du tiroir 2. Une telle caméra et les photographies qu'elle capture permettent notamment de faciliter ultérieurement pour un opérateur, l'opération consistant à retrouver et retirer une cassette 1. En effet, ces dernières peuvent avoir des couleurs différentes et, à l'aide de la photographie, ainsi que des coordonnées, l'opérateur peut alors plus rapidement cibler la cassette 1 en question qu'il recherche.

Ces photographies permettent aussi de constituer une preuve numérique de la présence à un moment donné d'une cassette 1, au cas où le référencement venait à être défaillant. Pour ce faire, chaque photographie peut être horodatée, voire certifiée, notamment à l'aide d'une signature numérique et/ou d'un certificat numérique.

Par ailleurs, la structure 5 permet de recevoir en partie inférieure au moins un tiroir 2, comme représenté sur les figures 1 et 2 selon le mode préférentiel de réalisation. Selon un autre mode de réalisation, plusieurs tiroirs 2 peuvent être positionnés à l'intérieur de la structure 5, lesdits tiroirs 2 étant alors positionnés côte à côte, dans un même plan. Selon un autre mode de réalisation envisagé, plusieurs tiroirs 2 peuvent être empilés, la détection et le référencement s'effectuant uniquement au niveau du tiroir 2 supérieur. Ensuite, une fois l'intégralité du tiroir 2 supérieur répertoriée, ce dernier peut être automatiquement décalé ou sorti de la structure 5, pour répéter l'opération avec le tiroir 2 situé juste en dessous, et ainsi de suite. Cette solution permet d'automatiser le référencement à plus grande échelle, notamment dans le cas d'une armoire avec au moins une pile de tiroirs 2.

Dans un cas comme dans l'autre, la structure 5 comprend en partie inférieure des moyens de réception de chaque tiroir 2. Ces moyens comprennent au moins un logement 60, conformé de manière à permettre l'insertion d'un tiroir 2, notamment par coulissement selon une translation horizontale ou sensiblement horizontale. Des moyens adaptés permettent de verrouiller ledit tiroir 2 au sein dudit logement 60, notamment pendant toute l'étape de référencement. Le verrouillage et le déverrouillage des tels moyens peuvent être effectués manuellement ou automatiquement, notamment pendant toute la durée de la détection et de la lecture, empêchant l'extraction involontaire du tiroir 2 en cours de traitement.

En outre, ladite structure 5 peut être recouverte d'une coque ou d'un carter 8 de protection, comme visible sur la figure 1, permettant de protéger et de limiter l'accès aux moyens qu'elle enferme. Cette coque 8 permet aussi de limiter la lumière provenant de l'extérieur, améliorant ainsi les performances des détection et lecture optiques.

L'invention concerne aussi le dispositif 9 de mise en œuvre du procédé selon l'invention, à savoir de détection et de référencement de la position bidimensionnelle d'au moins une cassette au sein d'un tiroir, ledit tiroir comportant des compartiments 22 répartis selon des colonnes 23 et des rangées 24.

Un tel dispositif 9 comprend :
- des moyens de réception dudit tiroir 2, ouvert en face supérieure ;
- des moyens de détection optique de la présence d'une cassette au sein de chaque compartiment 22, sous la forme d'une mire optique 3 ; et
- des moyens de lecture optique de données présentes sur chaque cassette détectée, sous la forme d'un scanner lumineux 4,
- ces moyens étant montés solidairement mobiles au-dessus dudit tiroir 2 et commandés en déplacement de manière à parcourir une trajectoire survolant chacun desdits compartiments 22.

Ledit dispositif 9 comprend aussi des moyens de référencement de la position de chaque cassette 1 détectée par l'intermédiaire des coordonnées de son compartiment 22, selon sa colonne 23 et sa rangée 24. Il comprend encore des moyens de gestion de la position référencée de chaque cassette 1 au sein dudit tiroir 2 et des moyens d'enregistrement de ladite position au sein dudit gestionnaire.

Ainsi, le système, constitué du procédé et du dispositif de détection et de référencement selon l'invention, permet d'automatiquement et systématiquement référencer et répertorier des cassettes 1, en leur assurant une traçabilité parfaite et un stockage adapté.

## Revendications

1. Procédé de détection et de référencement de la position bidimensionnelle au sein d'un tiroir (2) d'au moins une cassette (1) de conservation de prélèvements tissulaires et cellulaires, dans le domaine médical, ledit tiroir (2) comportant des compartiments (22) répartis selon des colonnes (23) et des rangées (24), dans lequel :
- on positionne au moins une cassette (1) enfichée la face supérieure tournée vers le haut au sein d'un compartiment (22) dudit tiroir (2) ;
- on effectue automatiquement, lors d'un premier passage, une détection systématique de tous les compartiments (22) par vérification de la présence d'une cassette (1) au sein de chacun desdits compartiments (22), la détection étant optique et consistant à déplacer automatiquement relativement par rapport au-dessus dudit tiroir (2) selon au moins une trajectoire passant par tous les compartiments (22) des moyens de détection et de lecture comprenant une mire optique (3) émettant un faisceau lumineux, notamment un faisceau laser, permettant de mesurer la distance entre son point d'émission et le point de réflexion, et un scanner lumineux (4) ;
- en cas de détection de la présence d'une cassette (1) au sein de son compartiment (22), on effectue une lecture, par le scanner lumineux (4), de données présentes sur ladite face supérieure et on identifie ladite cassette (1) ;
- on référence et on enregistre la position bidimensionnelle de ladite cassette (1) identifiée par l'intermédiaire des coordonnées de son compartiment (22), selon sa colonne (23) et sa rangée (24) ;
- en cas d'erreur de lecture par le scanner lumineux, on enregistre la position où l'erreur de lecture a été constatée,
**caractérisé en ce que** : :
- en cas d'erreur de lecture lors d'un premier passage, les moyens de détection et de lecture sont déplacés directement vers la position où l'erreur survenue a été constatée et préalablement enregistrée, sans opérer une nouvelle détection et/ou une lecture de tous les autres compartiments (22) où ces opérations se sont déroulées convenablement auparavant lors du premier passage, et la détection est réitérée directement à l'emplacement où l'erreur est survenue, s'abstenant de refaire l'intégralité du premier passage qui parcourt l'intégralité du tiroir.

2. Procédé selon la revendication 1, **caractérisé en ce que** la lecture est optique, lesdites données étant encodées sous forme d'un code à barres ou matriciel lu par le scanner lumineux (4).

3. Procédé selon l'une quelconque des revendications précédentes, **caractérisé en ce que** le référencement comprend une étape d'enregistrement de la position référencée en rapport avec les données de chaque cassette (1) au sein d'un gestionnaire.

4. Procédé selon l'une quelconque des revendications précédentes, **caractérisé en ce qu'**il consiste à effectuer plusieurs détections et lectures successives des compartiments (22) dudit tiroir (2).

## Patentansprüche

1. Verfahren zum Ermitteln und Referenzieren der zweidimensionalen Position in einem Schubfach (2) mindestens einer Kassette (1) zur Aufbewahrung von Gewebe- und Zellproben im medizinischen Bereich, wobei das Schubfach (2) Fächer (22) aufweist, die gemäß Spalten (23) und Reihen (24) verteilt sind, wobei:
- mindestens eine Kassette (1), mit der Oberseite nach oben zeigend eingesetzt in ein Fach (22) des Schubfachs (2) positioniert wird;
- bei einem ersten Durchgang eine systematische Ermittlung aller Fächer (22) durch Überprüfung des Vorhandenseins einer Kassette (1) in jedem der Fächer (22) automatisch durchgeführt wird, wobei die Bestimmung optisch ist und darin besteht, relativ in Bezug auf oberhalb des Schubfachs (2) gemäß mindestens einer Bahn, die durch alle Fächer (22) verläuft, Ermittlung- und Lesemittel automatisch zu verlagern, die eine optische Messlatte (3) umfassen, die einen Lichtstrahl sendet, insbesondere einen Laserstrahl, der erlaubt, den Abstand zwischen seinem Sendepunkt und dem Reflexionspunkt zu messen, und einen Lichtscanner (4);
- bei Ermittlung des Vorhandenseins einer Kassette (1) in ihrem Fach (22) durch den Lichtscanner (4) Daten abgelesen werden, die auf der Oberseite vorhanden sind und die Kassette (1) identifiziert wird;
- die zweidimensionale Position der identifizierten Kassette (1) anhand der Koordinaten ihres Fachs (22) gemäß ihrer Spalte (23) und ihrer Reihe (24) referenziert und registriert wird;
- bei einem Ablesefehler des Lichtscanners die Position registriert wird, wo der Ablesefehler festgestellt wurde,
**dadurch gekennzeichnet, dass**:
bei einem Ablesefehler bei einem ersten Durchgang die Ermittlungs- und Lesemittel direkt zu der Position verlagert werden, wo der aufgetretene Fehler festgestellt und zuvor registriert wurde, ohne eine neue Ermittlung und/oder Ablesung aller anderen Fächer (22) durchzuführen, wo diese Vorgänge zuvor beim ersten Durchgang korrekt durchgeführt wurden, und die Ermittlung direkt an der Stelle wiederholt wird, an der der Fehler aufgetreten war, bei Verzicht auf eine Wiederholung des gesamten ersten Durchgangs, der das gesamte Schubfach erfasst.

2. Verfahren nach Anspruch 1, **dadurch gekennzeichnet, dass** das Ablesen optisch ist, wobei die Daten in Form eines Strich- oder Matrixcodes codiert sind, der von dem Lichtscanner (4) abgelesen wird.

3. Verfahren nach einem der vorangehenden Ansprüche, **dadurch gekennzeichnet, dass** das Referenzieren einen Schritt des Registrierens der referenzierten Position bezüglich der Daten jeder Kassette (1) in einer Verwaltungseinrichtung umfasst.

4. Verfahren nach einem der vorangehenden Ansprüche, **dadurch gekennzeichnet, dass** es darin besteht, mehrere aufeinanderfolgende Ermittlungen und Ablesungen der Fächer (22) des Schubfachs (2) durchzuführen.

## Claims

1. Method of scanning and referencing the two-dimensional position within a drawer (2) of at least one cassette (1) for storing tissue and cellular samples, in the medical field, said drawer (2) comprising compartments (22) distributed in columns (23) and rows (24), in which:
- positioning at least one cassette (1) inserted with the upper surface facing upwards within a compartment (22) of said drawer (2);
- automatically performing, during a first pass, a systematic scanning of all the compartments (22) by checking the presence of a cassette (1) within each of said compartments (22), the scanning being optical and consistent in moving automatically and relatively to the top of said drawer (2) along at least one trajectory passing over all the compartments (22) by the scanning and reading means comprising an optical foccal bead (3) emitting a light beam, in particular a laser beam, making it possible to measure the distance between its point of emission and the point of reflection, and a light scanner (4);
- in the event of detection of the presence of a cassette (1) within its compartment (22), a reading is performed, by the light scanner (4), of data present on said upper surface and said cassette (1) is identified;
- the two-dimensional position of said cassette (1) as identified by means of the coordinates of its compartment (22), according to its column (23) and its row (24), is referenced and recorded.
- in the event of a reading error by the light scanner, the position where the reading error was observed is recorded,
**characterized in that**:
- in the event of a reading error during a first pass, the detection and reading means are moved directly to the position where the observed error occurred and was previously recorded, without performing a new scanning and/or reading of all the other compartments (22) where these operations have previously properly taken place during the first pass, and the scanning is repeated directly at the location where the error occurred, thus refraining from performing the entire first pass again and scanning the entire drawer.

2. Method according to claim 1, **characterized in that** the reading is optical, said data being encoded in the form of a bar code or matrix read by the light scanner (4).

3. Method according to any one of the preceding claims, **characterized in that** the referencing comprises a step of recording the referenced position in relation to the data of each cassette (1) within a database.

4. Method according to any one of the preceding claims, **characterized in that** it consists in performing several successive scans and readings of the compartments (22) of said drawer (2).
